(19) **Europäisches Patentamt**

**European Patent Office**

**Office européen des brevets**

(11) **EP 4 631 497 A1**

(12) **EUROPÄISCHE PATENTANMELDUNG**

(43) Veröffentlichungstag:
**15.10.2025 Patentblatt 2025/42**

(21) Anmeldenummer: **24169478.5**

(22) Anmeldetag: **10.04.2024**

(51) Internationale Patentklassifikation (IPC):
*A61K 9/00* (2006.01)    *A61K 8/14* (2006.01)
*A61K 9/127* (2025.01)    *A61K 31/05* (2006.01)
*A61K 31/12* (2006.01)    *A61K 31/352* (2006.01)
*A61K 31/7048* (2006.01)    *A61Q 19/00* (2006.01)
*A61K 47/69* (2017.01)

(52) Gemeinsame Patentklassifikation (CPC):
**A61K 31/352; A61K 8/14; A61K 8/738;**
**A61K 9/0014; A61K 9/127; A61K 31/05;**
**A61K 31/12; A61K 31/7048; A61K 47/6951;**
**A61Q 19/00;** A61K 2800/10

(84) Benannte Vertragsstaaten:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Benannte Erstreckungsstaaten:
**BA**
Benannte Validierungsstaaten:
**GE KH MA MD TN**

(71) Anmelder: **Evanium Healthcare GmbH**
**93053 Regensburg (DE)**

(72) Erfinder:
- **OFNER, David**
  **93047 Regensburg (DE)**
- **ROLKA, Felix**
  **93047 Regensburg (DE)**

(74) Vertreter: **Weickmann & Weickmann PartmbB**
**Postfach 860 820**
**81635 München (DE)**

(54) **LIPOSOMALE ZUSAMMENSETZUNG ZUR TOPISCHEN VERABREICHUNG**

(57)    Die Erfindung betrifft ein System aus beschichteten Liposomen, die mit einem Wirkstoff beladen sind, der in Cyclodextrin eingeschlossen ist. Außerdem wird deren Verwendung zur Verbesserung der Penetration schwerlöslicher Wirkstoffe in die Haut sowie ein Verfahren zur Herstellung der beschichteten Liposomen beschrieben.

EP 4 631 497 A1

## Beschreibung

Die Erfindung betrifft ein System aus beschichteten Liposomen, die mit einem Wirkstoff beladen sind, der in Cyclodextrin eingeschlossen ist. Außerdem wird deren Verwendung zur Verbesserung der Penetration schwerlöslicher Wirkstoffe in die Haut sowie ein Verfahren zur Herstellung der beschichteten Liposomen beschrieben.

## Hintergrund

Die topische Anwendung von Wirkstoffen bietet eine Vielzahl von Vorteilen. Durch die direkte Applikation am Zielort ermöglicht sie eine gezielte Behandlung, ohne den gesamten Körper zu beeinflussen. Dies reduziert das Risiko von systemischen Nebenwirkungen im Vergleich zur oralen oder parenteralen Verabreichung erheblich. Zudem sind topisch angewendete Wirkstoffe oft besser verträglich, da sie nicht den Verdauungstrakt passieren und somit Magen-Darm-Beschwerden verursachen können. Die Anwendung ist in der Regel einfach und erfordert keine speziellen Fähigkeiten oder Ausrüstungen, was sie für Patienten praktisch macht. Darüber hinaus minimiert die topische Anwendung die Menge des Wirkstoffs, die in den Blutkreislauf gelangt, was das Risiko von Wechselwirkungen mit anderen Medikamenten reduziert. Die Dosierung kann oft leicht angepasst werden, indem die Menge oder Häufigkeit der Anwendung variiert wird, um den individuellen Bedürfnissen des Patienten gerecht zu werden. Insgesamt bietet die topische Anwendung von Wirkstoffen eine effektive und schonende Möglichkeit zur Behandlung verschiedener Erkrankungen und Beschwerden.

Die Haut erfüllt eine essenzielle Schutzfunktion für den menschlichen Körper. Als äußerste Barriere bildet die Epidermis, die oberste Schicht der Haut, eine physikalische Barriere, die das Eindringen von Mikroorganismen, Chemikalien und anderen potenziell schädlichen Substanzen verhindert. Durch ihre dicht gepackten Zellen, Lipide und Proteine bietet die Epidermis einen effektiven Schutz vor äußeren Einflüssen.

Darüber hinaus produziert die Haut Talg, eine ölige Substanz, die die Haut vor Austrocknung schützt und eine saure Umgebung schafft, die das Wachstum von schädlichen Bakterien hemmt. Diese chemische Barriere trägt ebenfalls zur Abwehr von Krankheitserregern bei. Zusätzlich enthält die Haut antimikrobielle Peptide, die Infektionen bekämpfen können.

Die Haut dient auch als Immunbarriere, da sie eine Vielzahl von Immunzellen beherbergt, die Krankheitserreger erkennen und das Immunsystem aktivieren können, um Infektionen zu bekämpfen. Diese Immunzellen, wie Langerhans-Zellen, spielen eine wichtige Rolle bei der Abwehr von Infektionen und der Aufrechterhaltung der Hautgesundheit.

Insgesamt ist die Schutzfunktion der Haut von entscheidender Bedeutung, um den Körper vor äußeren Einflüssen zu schützen, die die Gesundheit und das Wohlbefinden beeinträchtigen könnten. Durch ihre verschiedenen Barriere- und Abwehrmechanismen trägt die Haut maßgeblich dazu bei, die Integrität des Körpers zu erhalten und das Risiko von Infektionen und anderen schädlichen Einflüssen zu minimieren.

Ein Nachteil dieser wichtigen Schutzfunktionen ist jedoch, dass sie auch kosmetische Wirkstoffe an der Hautoberfläche halten, für die ein tieferes Eindringen in die Haut wünschenswert wäre.

Obwohl die Haut eine effektive Barriere gegen das Eindringen von Fremdstoffen bildet, ist sie jedoch nicht undurchlässig. Einige Substanzen können durch die Haut hindurch diffundieren und in den Körper gelangen. Die Hautpermeation ist von besonderer Bedeutung in der Pharmazie und Kosmetikindustrie, da sie die Effektivität von topisch angewendeten Medikamenten, Cremes, Salben und Kosmetika beeinflusst. Ein tieferes Verständnis der Hautpermeation hilft dabei, die Wirksamkeit und Sicherheit von topischen Produkten zu verbessern und die gezielte Verabreichung von Wirkstoffen zu optimieren.

Die Hautpermeabilität von Wirkstoffen hängt von verschiedenen Faktoren ab, einschließlich der Molekülgröße, der Lipophilie (Fettlöslichkeit), der Ladung, der Löslichkeit in Wasser und anderen physikochemischen Eigenschaften. Generell können Lipophilie und eine geringe Molekülgröße die Hautpermeation erleichtern, während hydrophile und größere Moleküle oft eine geringere Permeabilität aufweisen. Darüber hinaus spielen die Eigenschaften des konkreten Wirkstoffs, wie Konzentration, Löslichkeit/Löslichkeitsgeschwindigkeit, Partikelgröße und Kristallinität eine wichtige Rolle. Weitere Faktoren wie Hautfeuchtigkeit, Temperatur, pH-Wert und Hautdicke können ebenfalls die Hautpermeation beeinflussen. Daher ist es bei der Entwicklung von topischen Produkten wichtig, die Hautpermeabilität der enthaltenen Wirkstoffe zu berücksichtigen und geeignete Strategien zur Verbesserung der Penetration einzusetzen.

Die Aufgabe der Erfindung war es daher, eine Möglichkeit zur Verbesserung der Hautpermeation kosmetischer Wirkstoffe zu entwickeln.

## Beschreibung

Überraschenderweise wurde gefunden, dass selbst schwerlösliche Wirkstoffe sehr effektiv in die Haut penetrieren, wenn Sie in ein spezielles System aus beschichteten Liposomen eingearbeitet wurden. Ein erster Aspekt der Erfindung betrifft daher eine wässrige Zusammensetzung, die Liposomen umfasst, die mit mindestens einem Wirkstoff beladen sind und an ihrer Außenseite eine Beschichtung aufweisen, die modifizierte Stärke umfasst, wobei der Wirkstoff mit einem alkylierten oder hydroxyalkylierten Derivat von β-Cyclodextrin assoziiert ist.

Als schwerlösliche Wirkstoffe werden im Sinne der Erfindung Wirkstoffe mit einer Löslichkeit bis 10mg/ml in reinem Wasser bei 20°C angesehen:

| Löslichkeitsklassifikation | Absolute Löslichkeit (dest. Wasser, 20°C) |
|---|---|
| Very Soluble | >1000mg/ml |
| Freely Soluble | 100-1000mg/ml |
| Soluble | 33-100mg/ml |
| Sparingly Soluble | 10-33mg/ml |
| Slightly Soluble | 1-10mg/ml |
| Very Slightly Soluble | 0,1-1mg/ml |
| Practically Insoluble | <0,1mg/ml |

Für entsprechende Wirkstoffe in der Kategorie "Practically Insoluble" sowie "Very Slightly Soluble" sowie im weiteren Sinne auch bis "Slightly Soluble", ist die effektive Gabe über topische Darreichungen problematisch.

Sowohl liposomale Systeme als auch Cyclodextrine werden häufig zur topischen Penetrationserhöhung eingesetzt. Liposomen weisen eine der Zellmembran ähnliche Lipiddoppelschichtstruktur auf, die einen Hohlraum umschließt. Sie werden häufig als Träger für Wirkstoffe verwendet, wobei sie in der Lage sind, hydrophobe Moleküle in ihrer Lipiddoppelschicht zu transportieren sowie hydrophile Wirkstoffe in ihren Hohlräumen aufzunehmen. Cyclodextrine sind cyclische Oligosaccharide, die aus α-1,4-glycosidisch verknüpften Glucosemolekülen zusammengesetzt sind. Dadurch weisen sie eine toroidale Struktur mit einem zentralen Hohlraum auf. Dieser innere Hohlraum ist hydrophob, während die äußere Oberfläche hydrophil ist. Der Hohlraum bietet Platz für die Aufnahme von hydrophoben Wirkstoffen, während die hydrophile Oberfläche die Löslichkeit begünstigt.

Insbesondere für schwerlösliche Wirkstoffe ergibt sich bei der Formulierung topischer Darreichungsformen das Problem des "Solubility-Permeability-Tradeoff", wobei die Löslichkeit des Wirkstoffes in der Matrix der Darreichungsform und damit die effektive Wirkstoffkonzentration treibende Kraft für die Penetration darstellt. Gleichzeitig wird bei zu hoher Löslichkeit und dementsprechend ungünstigem Verteilungskoeffizient zwischen Darreichungsform und Haut die Permeabilität und dadurch letztlich auch die Penetration vermindert, da der Wirkstoff in der Matrix zurückgehalten wird. Sollen schwerlösliche Wirkstoffe formuliert werden, gibt es hierzu entsprechend oftmals eine Abwägung, ob die Löslichkeit erhöht werden soll bei gleichzeitiger Verringerung der Permeabilität oder andersherum. Um diese Probleme zu überwinden, werden erfindungsgemäß die Effekte von Liposomen und Cyclodextrinen kombiniert, indem ein mit Cyclodextrin verbundener Wirkstoff in Liposomen eingelagert wird. Die Einbindung des Wirkstoffs in Cyclodextrin verbessert seine Löslichkeit in der Matrix, während über die Liposomen der gewünschte Transport durch die Haut wesentlich gesteigert werden kann.

Erfindungsgemäß werden hierzu alkylierte und hydroxyalkylierte Derivate von β-Cyclodextrin eingesetzt, bei diesen sind Hydroxylgruppen des β-Cyclodextrins unter Bildung von Alkyl- oder Hydroxyalkylether-Gruppen verethert. Viele hydrophobe Wirkstoffe gehen mit diesen β-Cyclodextrinderivaten Einschlusskomplexe ein, wodurch ihre Löslichkeit wesentlich verbessert werden kann. Als besonders geeignet hat sich für diesen Zweck Hydroxypropyl-β-Cyclodextrin erwiesen. Der Gewichtsanteil des β-Cyclodextrinderivats in einer erfindungsgemäßen Zusammensetzung beträgt 10-25 % w/w, bezogen auf die Gesamtzusammensetzung. Besonders vorteilhaft sind Konzentrationen im Bereich von 12,5-22,5 % w/w β-Cyclodextrinderivat, vorzugsweise 15-20 % w/w β-Cyclodextrinderivat.

Liposomen dienen erfindungsgemäß der Erhöhung der Permeabilität der Wirkstoffe, sodass sie die Barriereschicht der Haut überwinden und topisch verabreicht werden können. Die Doppelschicht der Liposomen umfasst ein oder mehrere Phospholipide, deren Anteil an der Gesamtzusammensetzung 1-4 % w/w beträgt. Besonders vorteilhaft sind Konzentrationen im Bereich von 1,5-3 % w/w, vorzugsweise 2-3 % w/w Phospholipide bezogen auf die Gesamtzusammensetzung.

In bevorzugten Ausführungsformen der Erfindung umfassen die Liposomen weiterhin ein oder mehrere Polyole. Diese sind vorzugsweise aus der Gruppe ausgewählt, die Butylenglycol, Pentylenglycol, Propylenglycol, Glycerin und Kombinationen davon umfasst. Der Gewichtsanteil der Polyole kann, bezogen auf die Gesamtzusammensetzung, insgesamt 2-20 % w/w betragen, bevorzugt 5-15 % w/w.

Überraschenderweise wurde in diesem Zusammenhang gefunden, dass die Permeabilität noch signifikant weiter verbessert werden kann, wenn die Liposomen mit einer äußeren Beschichtung versehen werden, die modifizierte Stärke umfasst. Der Gewichtsanteil der modifizierten Stärke beträgt bezogen auf die Gesamtzusammensetzung 0,5-1,5 % w/w.

Unter modifizierter Stärke werden erfindungsgemäß insbesondere Stärkeether-Derivate wie Hydroxypropylstärke und Stärkeester-Derivate wie Octenylsuccinatstärke verstanden, bei denen Hydroxygruppen der Glucose-Einheit der Stärke verethert bzw. verestert sind.

Wesentlich bei der Auswahl einer geeigneten modifizierten Stärke ist zum einen ihr Substitutionsgrad und zum anderen ihre Viskosität. Der Substitutionsgrad, angegeben in Gewichtsprozent der modifizierten Einheit, bezieht sich auf die Trockenmasse, und kann durch Titration ermittelt werden. Erfindungsgemäß verwendete Stärke weist vorzugsweise einen Substitutionsgrad von 0,1-10% w/w, bevorzugt 0,1-7%, weiter bevorzugt 0,5-5 % w/w oder 0,5-3 % w/w auf, bezogen auf die Trockenmasse. Die Viskosität der modifizierten Stärke kann mittels Brookfield LV Viskosimeter, Spindel LV1 als 10% w/w Lösung bei 20°C gemessen werden. Im Sinne der Erfindung werden ausschließlich Stärken mit einer geringen Viskosität von < 100 mPas genutzt, bevorzugt < 50 mPas, besonders bevorzugt < 25 mPas, am besten < 15 mPas 10%w/w Lösung bei 20°C. Modifizierte Stärken mit einer Viskosität in diesem Bereich sind besonders gut geeignet, um eine gleichmäßige Beschichtung zu erreichen, die zudem die Stabilität der Formulierung nicht beeinträchtigt. Bei höher viskosen Stärken ist die Ummantelung dagegen so stark ausgeprägt, dass es zu einer Instabilität der Formulierung kommt.

Der Polydispersibilitätsindex (PDI) der mit modifizierter Stärke beschichteten Liposomen sollte vorteilhafterweise bei < 0,3 liegen, da dies eine enge Partikelgrößenverteilung anzeigt. Bevorzugt ist ein PDI-Wert der beschichteten Liposomen von < 0,25 und besonders bevorzugt < 0,2. Bei zu langer (zu viskoser) Stärke oder zu hoher Stärkekonzentration kann ein unregelmäßiges Coating stattfinden, wodurch Partikel mit ungleichmäßiger Beschichtung erzeugt werden, wohingegen niedrigviskose Stärken zu geringen PDI-Werten führen. Die Partikelgröße der beschichteten Liposomen in einer erfindungsgemäßen Zusammensetzung beträgt etwa 100-200 nm, vorzugsweise 125-175 nm.

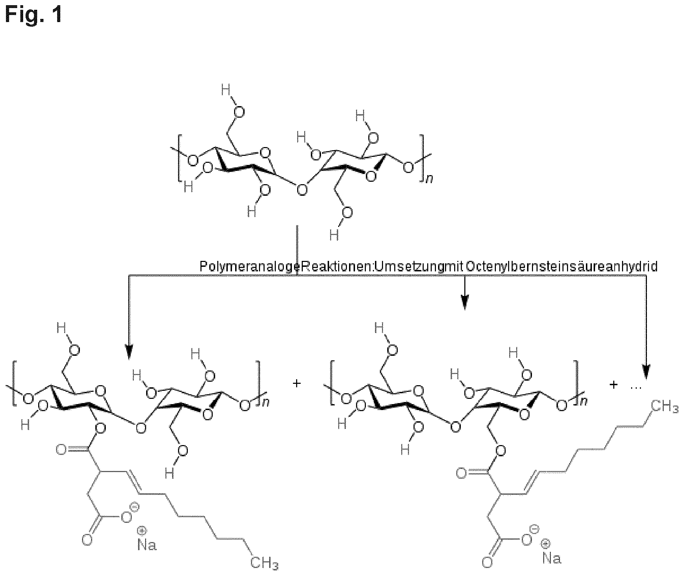

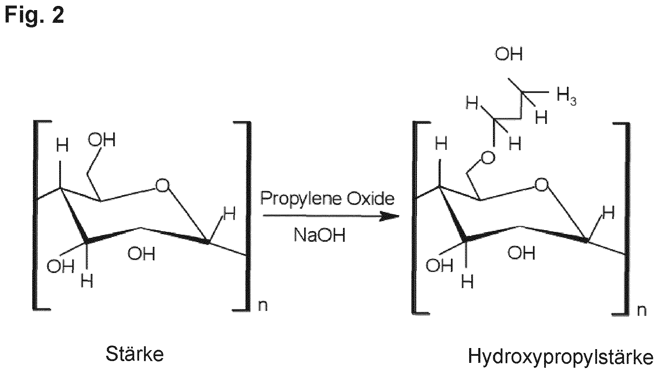

Modifizierte Stärke für das Coating der erfindungsgemäßen Liposome kann nach Gelatinisierung sowie Einkürzen der Kettenlängen durch enzymatische Behandlung oder Dextrinisierung (Fluidisierung) über eine Derivatisierungsbehandlung erhalten werden. In bevorzugten Ausführungsformen der Erfindung ist die modifizierte Stärke mit Octenylsuccinat-Gruppen oder Hydroxypropyl-Gruppen derivatisiert. Octenylsuccinat-Gruppen können durch Behandlung mit Octenylsuccinatanhydrid eingebracht werden, wobei die in Figur 1 gezeigte Struktur (Natriumoctenylsuccinatstärke) entsteht. Der Substitutionsgrad liegt bei Octenylsuccinatstärke vorzugsweise bei etwa 0,1-10% w/w, insbesondere 0,5-7 % w/w. Hydroxypropyl-Gruppen können analog durch Behandlung mit Propylenoxid eingebracht werden, wie in Figur 2 veranschaulicht. Der Substitutionsgrad liegt bei Hydroxypropylstärke vorzugsweise bei etwa 0,1-5 % w/w, insbesondere 0,5-5 % w/w. oder 0,5-3 % w/w.

Wirkstoffe, die mit den erfindungsgemäßen beschichteten Liposomen verkapselt werden können, sind insbesondere hydrophobe Wirkstoffe mit einer geringen Wasserlöslichkeit von 10mg/ml und weniger (in reinem Wasser bei 20°C). Besonders vorteilhaft ist die erfindungsgemäße Verkapselung für Wirkstoffe mit einer Wasserlöslichkeit von <1 mg/ml. Das Molekulargewicht der Wirkstoffe kann im Bereich 200 -1500 g/mol liegen. Die chemische Struktur der Wirkstoffe ist grundsätzlich beliebig wählbar, sofern sie zu einer nicht-kovalenten Wechselwirkung mit Cyclodextrin in der Lage sind. Es kann sich um natürliche oder synthetische Stoffe mit einer geringen Wasserlöslichkeit handeln. Bevorzugt sind vor allem schwerlösliche sekundäre Pflanzenstoffe und deren Glycoside wie Stilbenoide (Resveratrol, Oxyresveratrol, Pterostilben, Piceatannol, Piceid, Pinosylvin, Rhaponticin, Desoxyrhaponticin, Gnetin, Rhapontigenin, Isorhapontigenin, Isorhapontin, Hopeaphenol), Flavonoide (Flavonole, Flavanone, Flavanole, Isoflavone, Flavonolignane), Di-, Sesqui- und Triterpene, Chalcone (Xanthohumol, Phloretin, Butein, Flavokavain, Hesperetin- bzw. Naringenin-Chalcon), Curcuminoide, Lignane, Hydrochinonderivate (Thymochinon), Zimtsäurederivate (Ferulasäure, Cumarinsäure, Chlorogensäure), Steroide, Xanthone und Anthraquinone, Carotinoide.

Darüber hinaus eignet sich das System für schwerlösliche Wirkstoffe aus den Klassen Analgetika und Lokalanästhetika (wie z.B. Lidocain, Benzocain), Antibiotika (wie z.B. Neomycin, Erythromycin, Mupirocincin), Antimykotika (wie z.B. Clotrimazol, Ketoconazol, Terbinafin), Kortikosteroide, Antipsoriatika (wie z.B. Calcipotriol), Antivirale Mittel (wie z.B. Acyclovir), Entzündungshemmende Mittel (wie z.B. Diclofenac, Ibuprofen), Immunmodulatoren (wie z.B. Tacrolimus, Pimecrolimus), Antioxidantien (wie z.B. Tocopherole), Antihistaminika (wie z.B. Diphenhydramin, Loratadin), Antiacne-Mittel und Retinoide, Pigmentmodulatoren wie z.B. (Kojisäure).

Der Gewichtsanteil des Wirkstoffs in einer erfindungsgemäßen Zusammensetzung liegt bei 0,1-3 % w/w. Besonders vorteilhaft sind Wirkstoffbeladungen im Bereich von 0,25-2 % w/w und insbesondere 0,5-1,5 % w/w Wirkstoff bezogen auf die Gesamtzusammensetzung.

Die erfindungsgemäße Zusammensetzung ist aufgrund der verbesserten Permeabilität besonders gut für die topische Verabreichung geeignet. Sie kann beispielsweise in Form einer Creme, als Salbe, Serum oder Hydrogel vorliegen.

Neben den beschichteten Liposomen kann eine erfindungsgemäße Zusammensetzung weitere übliche Bestandteile einer für die topische Verabreichung vorgesehenen Formulierung umfassen.

Die erfindungsgemäße Zusammensetzung kann als pharmazeutische oder kosmetische Zusammensetzung formuliert werden. In einer bevorzugten Ausführungsform ist die erfindungsgemäße Zusammensetzung für die kosmetische Anwendung vorgesehen.

Ein weiterer Aspekt der Erfindung betrifft ein Verfahren zur Herstellung einer wässrigen Zusammensetzung, die beschichtete Liposomen umfasst, die mit alkyliertem oder hydroxyalkyliertem β-Cyclodextrin und mindestens einem Wirkstoff beladen sind, umfassend die Schritte:

a) Herstellen einer wässrigen Lösung von alkyliertem oder hydroxyalkyliertem β-Cyclodextrin, insbesondere Hydroxypropyl-β-Cyclodextrin und mindestens einem Wirkstoff, um den Wirkstoff mit dem Cyclodextrinderivat zu assoziieren, vorzugsweise um einen Einschlusskomplex des Wirkstoffs in dem Cyclodextrinderivat zu bilden,
b) Bilden von Liposomen, die einen Hohlraum umschließen in dem der mit dem Cyclodextrinderivat assoziierte Wirkstoff, insbesondere der Einschlusskomplex des Wirkstoffs in Hydroxypropyl-β-Cyclodextrin, eingebettet ist, und
c) Beschichten der Liposomen an ihrer Außenseite mit einer Beschichtung, die modifizierte Stärke umfasst.

Bei der Herstellung einer wässrigen Lösung in Schritt a) erfolgt eine Assoziierung des Wirkstoffs mit dem Cyclodextrinderivat, insbesondere Hydroxypropyl-β-Cyclodextrin. Hierzu kann eine wässrige Lösung des Cyclodextrinderivats vorgelegt und anschließend Wirkstoff zugegeben werden. Durch die Assoziierung mit dem Cyclodextrinderivat wird die Löslichkeit hydrophober Wirkstoffe, die allein schlecht wasserlöslich sind, wesentlich verbessert. Das Verfahren eignet sich besonders für die vorstehend im Zusammenhang mit einer erfindungsgemäßen Zusammensetzung beschriebenen Wirkstoffe. Durch Rühren und Erwärmen kann die Auflösung außerdem weiter begünstigt werden.

Unter einer Assoziierung wird im Sinne der Erfindung eine nicht-kovalente Wechselwirkung des Wirkstoffs mit dem Cyclodextrinderivat verstanden, wobei der Wirkstoff ganz oder teilweise in den Hohlraum des Cyclodextrinderivats aufgenommen wird. Vorzugsweise wird der Wirkstoff dabei unter Bildung eines Einschlusskomplexes in das Cyclodextrinderivat eingelagert. 2-Hydroxypropyl-β-Cyclodextrin ist hierzu besonders gut geeignet. Das Gewichtsverhältnis von Cyclodextrin und Wirkstoff wird so gewählt, dass der Wirkstoff möglichst vollständig in das Cyclodextrin aufgenommen wird. Vorteilhafterweise liegt der Gewichtsanteil des Wirkstoffs bei etwa 0,1-3 % w/w und der Gewichtsanteil des β-Cyclodextrinderivats bei etwa 10-25 % w/w, jeweils bezogen auf die Gesamtzusammensetzung,

Im anschließenden Schritt b) werden der in Schritt a) erhaltenen Lösung Liposom-bildende Moleküle zugegeben, die sich in der wässrigen Lösung zu einer doppelschichtigen Membranhülle anordnen, welche den mit Cyclodextrin assoziierten Wirkstoff umschließt. Hierzu können übliche Methoden zur Herstellung von Liposomen eingesetzt werden, die im Fachbereich bekannt sind. Geeignete Liposom-bildende Moleküle sind amphiphile Lipide, wie insbesondere Phospholipide. Die Menge der Phospholipide wird vorzugsweise so gewählt, dass ihr Gewichtsanteil an der Gesamtzusammensetzung etwa 1-4 % w/w beträgt. Besonders geeignet sind Konzentrationen von 1-5 % w/w oder 2-3 % w/w an Phospholipiden, bezogen auf die Gesamtzusammensetzung. Weiterhin können ein oder mehrere Polyole zugegeben werden, die vorzugsweise aus der Gruppe ausgewählt sind, die Butylenglycol, Pentylenglycol, Propylenglycol, Glycerin und Kombinationen davon umfasst. Der Gewichtsanteil der Polyole kann so gewählt werden, dass er, bezogen auf die Gesamtzusammensetzung, insgesamt 2-20 % w/w, bevorzugt 5-15 % w/w beträgt.

Die erhaltenen beladenen Liposomen werden dann in Schritt c) an ihrer Außenseite mit einer Beschichtung versehen, die modifizierte Stärke umfasst. Hierzu werden die Liposomen mit einer Stärkelösung in Kontakt gebracht. Vorteilhafterweise kann dabei direkt die in Schritt b) erhaltene Lösung der Liposomen eingesetzt werden, ohne dass eine vorherige Aufreinigung oder Isolierung erforderlich ist. Die Ausbildung einer Stärkebeschichtung erfolgt unter Ultraschall- oder Hochdruckhomogenisation, wobei der Druck im Allgemeinen niedriger liegt als bei der vorausgehenden Liposombildung. Dadurch wird erreicht, dass die zuvor gebildeten Liposomen erhalten bleiben und keine Liposom-Neubildung erfolgt. Vorzugsweise liegt der Druck unter 1000 bar, bevorzugt unter 800 bar, besonders bevorzugt im Bereich von etwa 400-600 bar.

Die Stärkemenge wird bezogen auf die Gesamtmasse der Zubereitung so gewählt, dass sie im Bereich 0,5-1,5% w/w liegt. Diese Mengen sind für die Beschichtung der Liposomen optimal, da hier ein leichter Anstieg des Partikeldurchmessers (Z-Average) zu sehen ist, was in Kombination mit einem leichten Zetapotential-Sprung auf ein erfolgreiches Coating hinweist. Gleichzeitig befindet sich der Polydispersivitätsindex (PDI) bei diesen Stärkekonzentrationen noch unter 0,3, vorzugsweise unter 0,25 oder besonders bevorzugt unter 0,2, wodurch ein gleichmäßiges Coating gewährleistet werden kann.

Geeignete Stärken für die Verwendung in dem erfindungsgemäßen Verfahren sind die vorstehend im Zusammenhang mit einer Liposomen-Zusammensetzung beschriebenen modifizierten Stärken.

Abhängig von einer gewünschten Verwendung der beschichteten Liposomen können sie zu einer Zusammensetzung für die topische Verabreichung weiterverarbeitet werden. Hierzu kann die Zusammensetzung beispielsweise als Creme, Salbe, Serum oder Hydrogel formuliert und gegebenenfalls mit weiteren Bestandteilen kombiniert werden. Besonders gut eignet sich die liposomale Zusammensetzung für den Einsatz in Öl-in-Wasser Systemen.

Typische weitere Bestandteile in solchen Systemen sind neben Wasser als Hauptlösemittel Öle und Fette (wie z.B. Mineralöl, Silikonöle, Pflanzenöle wie Jojobaöl oder Sonnenblumenöl Jojobaöl, Mandelöl, Arganöl, Kokosnussöl, Olivenöl, Sonnenblumenöl, Traubenkernöl, Avocadoöl, Sheabutter, Kakaobutter, Macadamianussöl, Hagebuttenkernöl, Nachtkerzenöl, Tamanuöl, Sesamöl und Wachse wie Bienenwachs, Wollwachs oder Paraffin), Emulgatoren (wie z.B.

Lecithin, Cetylalkohol, Stearylalkohol, Polysorbat 80, Ceteareth, Glycerylstearat, Sorbitanoleat, Glycerinmonostearat, Polyglyceryl-3-Methylglucose Distearat, PEG-40 Stearat, Polyglyceryl-6 Distearat, Cetearylglucosid, Steareth-2, Steareth-20, Behenylalkohol, Alkylglucoside) eingesetzt, sowie Konservierungsmittel (z.B. Phenoxyethanol, Benzoesäure, Ethylhexylglycerin, Parabene, Benzylalkohol, Benzylalkohol, Chlorphenesin, Diazolidinylharnstoff, DMDM Hydantoin, Imidazolidinylharnstoff, Methylchloroisothiazolinon, Methylisothiazolinon, Kaliumsorbat, Natriumbenzoat, Caprylylglykol, Triclosan), Feuchthaltemittel (wie z.B. Glycerin, Hyaluronsäure, Propylenglykol, Sorbitol, Butylenglykol, Panthenol, Sodium PCA, Urea, Glycolsäure, Milchsäure) und Verdickungsmittel (wie z.B. Carbomere, Xanthan-Gummi, Cellulose-Derivate, Acrylate, Bentonit, Kollagen, Guar-Gummi, Alginsäure, Carrageen, Silikone), Duftstoffe (wie z.B. Benzylalkohol, Limonen, Linalool, Citronellol, Geraniol, Eugenol, Citral, Farnesol, Alpha-Isomethyl-Ionon, Hydroxycitronellal, Coumarin, Hexylzimt, Benzylbenzoat, Amyl-Cinnamal, Benzylsalicylat), Farbstoffe (wie z.B. Eisenoxide (z.B. CI 77491, CI 77492, CI 77499), Titandioxid (CI 77891), Ultramarinblau (CI 77007), Carmine (CI 75470), Chromoxide (CI 77288), FD&C-Farbstoffe (z.B. FD&C Gelb Nr. 5 - CI 19140, FD&C Blau Nr. 1 - CI 42090), D&C-Farbstoffe (z.B. D&C Rot Nr. 27 - CI 45410, D&C Rot Nr. 6 - CI 15850), Mica (CI 77019), Tin Oxide (CI 77861)), pH Regulatoren (wie z.B. Natriumhydroxid, Kaliumhydroxid, Zitronensäure, Natriumcitrat, Dinatriumphosphat, Phosphorsäure, Natriumbicarbonat), Antioxidatien (wie z.B. Vitamin E (Tocopherol), Vitamin C (Ascorbinsäure), Coenzym Q10 (Ubichinon), Resveratrol, Grüner Tee Extrakt, Traubenkernextrakt (OPC), Niacinamid (Vitamin B3), Ferulasäure, Alpha-Liponsäure, Glutathion, Astaxanthin), UV-Filter (wie z.B. Avobenzon, Octocrylen, Octinoxat (Octylmethoxycinnamat), Octisalat (Octylsalicylat), Oxybenzon (Benzophenon-3), Homosalat, Mexoryl SX (Terephthalylidene Dicamphorsulfonsäure), Mexoryl XL (Drometrizol Trisiloxan), Tinosorb S (Bis-Ethylhexyloxyphenol Methoxyphenyl Triazin), Tinosorb M (Bis-Ethylhexyl Hydroxybenzoyl Hexyl Benzoat), Uvinul A Plus (Diethylamino Hydroxybenzoyl Hexyl Benzoat), Uvinul T 150 (Ethylhexyl Triazone)).

Die nachfolgenden Beispiele und die Figuren sollen die Erfindung weiter veranschaulichen. Die Erfindung ist jedoch nicht auf diese konkreten Beispiele beschränkt, sondern sie erstreckt sich auf den gesamten Umfang der Ansprüche.

**Figuren**

**Fig.1**   Reaktionsschema der Derivatisierung von Stärke mit Octenylsuccinatanhydrid

**Fig. 2**   Reaktionsschema der Derivatisierung von Stärke mit Propylenoxid

**Beispiele**

Zur Bestimmung einer optimalen Formulierung für die Hautpenetration wurden zunächst die Stärken charakterisiert, sowie Formulierungen enthaltend die schwerlöslichen Wirkstoffe Taxifolin, Phloretin, Naringin und Resveratrol hergestellt. Nach abschließender Charakterisierung wurde die Hautpenetration mithilfe der Franz-Diffusionszelle in verschiedenen Darreichungsformen (Creme, Hydrogel) ermittelt.

**1. Charakterisierung der Stärken (Viskosität)**

Verschiedene modifizierte Stärken (Natriumoctenylsuccinat- & Hydroxypropylstärken) wurden auf ihre Viskosität untersucht. Hierzu wurden die getrockneten Stärken auf einem Magnetrührer gelöst (20g Stärke + 180ml dest. Wasser), um eine 10% w/w Lösung herzustellen. Diese Lösung wurde anschließend mittels Brookfield DVPlus Viskosimeter (DVPLLV) bei 20°C vermessen.

*10% w/w Natriumoctenylsuccinatstärken:*

| Stärke/kommerzieller Name | Viskosität bei 20°C [mPas] | Spindel |
|---|---|---|
| Hi-Caps 100 (Ingredion) | 2-5 | Spindle LV-1 |
| Cleargum CO03 (Roquette) | 3-8 | Spindle LV-1 |
| Cleargum CO 01 (Roquette) | 5-10 | Spindle LV-1 |
| Purity Gum 2000 (Ingredion) | 6-11 | Spindle LV-1 |

(fortgesetzt)

| Stärke/kommerzieller Name | Viskosität bei 20°C [mPas] | Spindel |
|---|---|---|
| Purity Gum Ultra (Ingredion) | 50-75 | Spindle LV-1 |
| N-Creamer 2230 (Ingredion) | 8.000 - 15.000 | Spindle LV-1 |

*10% w/w Hydroxypropylstärken:*

| Stärke/kommerzieller Name | Viskosität bei 20°C [mPas] | Spindel |
|---|---|---|
| Lycoat RS 780 (Roquette) | 8-13 | Spindle LV-1 |
| Lycoat RS 720 (Roquette) | 25-50 | Spindle LV-1 |

### 2. Wirkstoffformulierungen mit Taxifolin

Aufgrund der geringen Wasserlöslichkeit (<1mg/ml bei 25°C) sowie der schlechten Hautpenetration eignet sich Taxifolin sehr gut als Wirkstoff zur Penetrationserhöhung durch die Erfindung.

#### 2.1 Material und Probenherstellung

**Material:** Taxifolinreicher Lächenextrakt (Lavitol 98% Taxifolin, Ametis JSC), Hydroxypropyl-$\beta$-Cyclodextrin (Beaute CD 110, Roquette), Vorgefertigte Liposomen (Natipide Eco, Lipoid), Natriumoctenylsuccinatstärke (Hi-CAPS 100, Cleargum CO 03, Cleargum CO 01, Purity Gum 2000, Purity Gum Ultra, N-Creamer 2230), Hydroxypropylstärken (Lycoat RS 780, Lycoat RS 720), Pentylene Glycol (Dermosoft Pentiol Eco, Evonik), HPLC Grade Wasser.
Die Gesamtzusammensetzung im Sinne der Erfindung ist wie folgt:

| Wirkstoff | Konzentration w/w |
|---|---|
| Taxifolin | 1% |
| Hydroxypropyl-$\beta$-Cyclodextrin | 20% |
| Natipide Eco<br>-davon Phospholipide berechnet als PC | 10%<br>2% |
| Modifizierte Stärke 20% w/w Lösung<br>-davon modifizierte Stärke | 5%<br>1% |
| Pentylene Glycol | 5% |
| Wasser | 59% |

Für die Zubereitung (25g gesamt) wurden zunächst 5g Hydroxypropyl-$\beta$-Cyclodextrin in 14,75g Wasser unter Rühren (300rpm, 25°C) vollständig gelöst. Hierzu wurden anschließend 0,25g Taxifolin gegeben und unter identischen Bedingungen gerührt, bis der Wirkstoff gelöst war. Zu dieser Cyclodextrin/Taxifolinlösung wurden 2,5g Natipide Eco und 1,25g Pentylene Glycol gegeben und für 30min bei 25°C und 300rpm gerührt, bis das Produkt homogen war. Hiernach wurde eine Probe zur Messung der Partikelgröße und dem Zetapotential genommen.
Anschließend wurden 1,25g einer 20%igen Stärkelösung (6 verschiedene Natriumoctenylsuccinatstärken [Hi-CAPS 100, Cleargum CO 03, Cleargum CO 01, Purity Gum 2000, Purity Gum Ultra, N-Creamer 2230] sowie 2 verschiedene Hydroxypropylstärken [(Lycoat RS 780, Lycoat RS 720]) unter Rühren hinzugegeben und die Lösung mittels Hochdruckhomogenisator (GEA, PandaPlus 1000) bei 450bar in 3 Zyklen homogenisiert. Von dem fertigen Produkt wurden ebenfalls Proben zur Messung der Partikelgröße und des Zetapotentials genommen.

#### 2.2 Charakterisierung der Proben (Partikelgröße und Zetapotential)

Die entnommenen Proben wurden mittels Photonenkorrelationsspektroskopie auf Partikelgröße (Z-Average) sowie Polydispersibilitätsindex (PDI) untersucht. Darüber hinaus wurde das Zeta-Potential gemessen. Für die Messungen wurde der Malvern Zetasizer Lab Red Label (90°C Lichtstreuungswinkel) genutzt, die Proben wurden vor Messung 1:100 mit gefiltertem, HPLC Grade Wasser verdünnt.

Ergebnisse vor Stärkezugabe:

| Produktname | Z-Average [nm] | PDI | Zeta Potential [mV] |
|---|---|---|---|
| Taxifolinformulierung vor Stärkezugabe | 132 | 0,126 | -45,6 |

Ergebnisse nach Stärkezugabe und Homogenisation:

| Samplename | Z-Average [nm] | PDI | Zeta Potential [mV] |
|---|---|---|---|
| Taxifolinformulierung + HI-CAP 100 | 146 | 0,153 | -36,7 |
| Taxifolinformulierung + Cleargum CO 03 | 142 | 0,146 | -38,9 |
| Taxifolinformulierung + Cleargum CO 01 | 152 | 0,148 | -37,4 |
| Taxifolinformulierung + Purity Gum 2000 | 158 | 0,162 | -32,6 |
| Taxifolinformulierung + Purity Gum Ultra | 228 | 0,233 | -25,8 |
| Taxifolinformulierung + N-Creamer 2230 | 453 | 0,466 | -11,8 |
| Taxifolinformulierung + Lycoat RS 780 | 153 | 0,151 | -35,4 |
| Taxifolinformulierung + Lycoat RS 720 | 210 | 0,194 | -28,3 |

Als stabil gelten Formulierungen mit mindestens +/-30mV, insofern sind insbesondere die niedrigviskosen Stärken geeignet. Ein Zeta-Potential Shift in den neutralen Bereich ist grundsätzlich durch das Stärkecoating zu erwarten und zeigt ebenso wie eine Zunahme des Z-Average die Ummantelung der Partikel an. Darüber hinaus wird ein vollständiges Coating durch eine unimodale Verteilung des Zetapotentials angezeigt, welches nach in Kontakt bringen der Liposomen mit der Stärkelösung und Behandlung mittels Ultraschall/Hochdruckhomogenisation erhalten wird. Allerdings ist dies bei den mittel- bis hochviskosen Stärken (Purity Gum Ultra, Lycoat RS 720, N-Creamer 2230) so stark ausgeprägt, dass es zu einer Instabilität der Formulierung kommt, während die niedrigviskosen, modifizierten Stärken ein gleichmäßiges Coating bilden und die Formulierung stabil bleibt.

Ebenso ist ein PDI (Polydispersibilitätsindex) von <0,2 wünschenswert, da dies eine enge Partikelgrößenverteilung anzeigt. Bei zu langer (zu viskoser) Stärke oder zu hoher Stärkekonzentration kann ein unregelmäßiges Coating stattfinden, wodurch Partikel mit ungleichmäßigem Coating erzeugt werden, wohingegen niedrigviskose Stärken zu geringen PDI Werten führen.

### 2.3 Einfluss der Stärkekonzentration auf Partikelgröße und Zetapotential

Um die Auswirkungen verschiedener Konzentrationen modifizierter Stärke auf das System bzw. das Coatingverhalten ermitteln zu können, wurden Wirkstoffformulierungen entsprechend 3.2.1. hergestellt, allerdings wurde statt gesamt nur 1% modifizierte Stärke zusätzlich die Konzentrationen 0,05%, 0,1% 0,5% 1,5%, 3% 6% getestet. Als Stärken wurden entweder Cleargum CO 03 oder Lycoat RS 780 genutzt. Die Partikelgrößen bzw. das Zetapotential der Formulierungen wurden entsprechend der Methode 3.2.2. gemessen:

Natriumstärkeoctenylsuccinat (Cleargum CO 03):

| Samplename | Z-Average [nm] | PDI | Zeta Potential [mV] |
|---|---|---|---|
| Taxifolinformulierung + 0,05% mod. Stärke | 131 | 0,123 | -45,9 |
| Taxifolinformulierung + 0,1% mod. Stärke | 132 | 0,133 | -47,8 |
| Taxifolinformulierung + 0,5% mod. Stärke | 140 | 0,144 | -41,3 |
| Taxifolinformulierung + 1% mod. Stärke | 142 | 0,146 | -36,9 |
| Taxifolinformulierung + 1,5% mod Stärke | 148 | 0,151 | -38,9 |
| Taxifolinformulierung + 3% mod. Stärke | 233 | 0,289 | -20,1 |
| Taxifolinformulierung + 6% mod. Stärke | 310 | 0,411 | -18,3 |

Hydroxypropylstärke (Lycoat RS 780):

| Samplename | Z-Average [nm] | PDI | Zeta Potential [mV] |
|---|---|---|---|
| Taxifolinformulierung + 0,05% mod. Stärke | 133 | 0,120 | -46,3 |
| Taxifolinformulierung + 0,1% mod. Stärke | 135 | 0,123 | -45,2 |
| Taxifolinformulierung + 0,5% mod. Stärke | 148 | 0,151 | -37,7 |
| Taxifolinformulierung + 1% mod. Stärke | 153 | 0,144 | -32,6 |
| Taxifolinformulierung + 1,5% mod Stärke | 155 | 0,160 | -34,5 |
| Taxifolinformulierung + 3% mod. Stärke | 311 | 0,252 | -26,1 |
| Taxifolinformulierung + 6% mod. Stärke | 363 | 0,339 | -22,5 |

Insbesondere Stärkekonzentrationen im Bereich 0,5-1,5% sind optimal, da hier ein leichter Anstieg des Partikeldurchmessers Z-Average zu sehen ist, was in Kombination mit einem leichten Zetapotential Sprung auf ein erfolgreiches Coating hinweist. Gleichzeitig befindet sich der PDI der gecoateten Liposomen bei diesen Stärkekonzentrationen noch unter 0,2, wodurch ein gleichmäßiges Coating gewährleistet werden konnte.

### 2.4 Herstellung von Kontrollproben (HP-ß-Cyclodextrin bzw. Phospholipid)

Zur Kontrolle bei den Penetrationsversuchen wurden darüber hinaus Wirkstoffformulierungen mit Taxifolin und I) nur Hydroxypropyl-$\beta$-Cyclodextrin oder II) nur Natipide Eco/Phospholipide hergestellt. Die Gesamtzusammensetzung ist wie folgt:

HP-$\beta$-Cyclodextrin/Taxifolin

| Wirkstoff | Konzentration w/w |
|---|---|
| Taxifolin | 1% |
| Hydroxypropyl-$\beta$-Cyclodextrin | 20% |
| Wasser | 79% |

Phospholipid/Taxifolin

| Wirkstoff | Konzentration w/w |
|---|---|
| Taxifolin | 1% |
| Natipide Eco | 10% |
| -davon Phospholipide berechnet als PC | 2% |
| Wasser | 89% |

Die Herstellung geschah gemäß der vorher beschrieben Formulierung, zunächst wurde Taxifolin, Wasser und die dritte Komponente (entweder Cyclodextrin oder Phospholipid) für 30min bei 25°C und 300rpm gerührt. Anschließend wurde die Lösung mittels Hochdruckhomogenisator (GEA, PandaPlus 1000) bei 450 bar in 3 Zyklen homogenisiert. Die Proben wurden ebenfalls auf Partikelgröße und Zetapotential untersucht:

| Produktname | Z-Average [nm] | PDI | Zeta Potential [mV] |
|---|---|---|---|
| HP-$\beta$-Cyclodextrin /Taxifolin | 22 | 0,117 | -0,2 |
| Phospholipid/Taxifolin | 138 | 0,138 | -42,1 |

## 2.5 Herstellung Cremeformulierung für Penetrationsversuche

Die Wirkstoffformulierungen nach Stärkezugabe/Homogenisation bzw. die Kontrollformulierungen wurden anschließend für die Penetrationsversuche in eine Cremeformulierung eingearbeitet. Darüber hinaus wurden auch zum Vergleich Cremeformulierungen mit reinem Taxifolin hergestellt.

Als Basis für die Creme wurde anionische hydrophile Creme SR genutzt, welche folgende Inhaltsstoffe enthält:

| Wirkstoff | Konzentration w/w |
|---|---|
| Emulgierender Cetylstearylalkohol (Typ A) | 21% |
| 2-Ethylhexyllaurat | 10 |
| Glycerol 85% | 4,25% |
| Kaliumsorbat | 0,14% |
| Citronensäure | 0,07% |
| Gereinigtes Wasser | 64,54% |

Zur Herstellung der finalen Formulierung wurde zunächst der Wasseranteil sowie der native Wirkstoff bzw. die wirkstoffhaltige Formulierung gemischt und diese anschließend in einem Mörser mit der Cremebasis homogenisiert. Es wurden Cremes enthaltend 2% Taxifolin nativ sowie enthaltend 0,1% bzw. 0,05% Taxifolin in Form der wie oben beschrieben hergestellten erfinderischen Wirkstoffformulierung mit verschiedenen Stärken bzw. in Form der Kontrollformulierungen mit entweder nur HP-$\beta$-Cyclodextrin oder nur Phospholipid hergestellt.

Die Zusammensetzung der Cremeformulierung enthaltend natives Taxifolin war wie folgt:

| Wirkstoff | Konzentration w/w |
|---|---|
| Taxifolin nativ | 2% |
| Anionische hydrophile Creme SR | 50% |
| Wasser | 48% |

Die Zusammensetzung der Cremeformulierung enthaltend 0,1% Taxifolin als Wirkstoffformulierung war wie folgt:

| Wirkstoff | Konzentration w/w |
|---|---|
| Taxifolinformulierung | 10% |

(fortgesetzt)

| Wirkstoff | Konzentration w/w |
|---|---|
| -davon Taxifolin | 0,1% |
| Anionische hydrophile Creme SR | 50% |
| Wasser | 40% |

Die Zusammensetzung der Cremeformulierung enthaltend 0,05% Taxifolin als Wirkstoffformulierung war wie folgt:

| Wirkstoff | Konzentration w/w |
|---|---|
| Taxifolinformulierung<br>-davon Taxifolin | 5%<br>0,05% |
| Anionische hydrophile Creme SR | 50% |
| Wasser | 45% |

Es wurde jeweils eine Creme für eine erfinderische Formulierung enthaltend jede der oben besprochenen Stärken (HI-CAP 100, Cleargum CO 03, Cleargum CO 01, Purity Gum 2000, Purity Gum Ultra, N-Creamer 2230, Lycoat RS 780, Lycoat RS 720) mit 0,1% bzw. 0,05% Taxifolinkonzentration sowie die Kontrollcremes hergestellt.

### 2.6 Penetrationsversuche mittels Franz-Diffusionszelle

Die Cremeformulierungen wurden abschließend zur Ermittlung der Hautpenetration in einer vertikalen Braunglas Franz-Diffusionszelle (PermeaGear 6 Positionen Franzzellenrührer enthaltend 6 ummantelte Franzzellen 11.28mm Durchmesser, 8ml Volumen, 1.00 cm$^2$ Membranfläche) untersucht. Hierzu wurde das Wasserbad auf 32°C beheizt, das untere Kompartment bestand aus 0,1% Tween80 in HPLC Grade Wasser, als Membran wurde die PermeaPad Skin Membran genutzt. Nach Auftragen der Cremeformulierung in das obere Kompartment wurde der Samplingarm und das obere Kompartment mit Parafilm abgedichtet. Es wurden Versuche in Triplikaten durchgeführt und die Durchschnittswerte berechnet.

Es wurden mittels einer Spritze nach 1h, 2h, 3h, 4h und 24h jeweils 300μl aus dem unteren Kompartment entnommen und mit frischer, vortemperierter Lösung (0,1% Tween80 in HPLC Grade Wasser) ersetzt. Die Samples wurden unverdünnt mittels HPLC analysiert (Agilent 1260 Infinity II DAD). Die Konzentration C im unteren Kompartment in μg/ml Taxifolin (1h, 2h, 3h, 4h und 24h) wurden kumuliert berechnet (unter Berücksichtigung der jeweils entnommenen 300μl).

Besonders relevant ist hierbei die kumulierte Endkonzentration im unteren Kompartment nach 24h, da hiermit die apparente Permeabilität (Papp) berechnet werden kann. Dieser Wert ist ein konzentrationsunabhängiger Maßstab für Permeabilität, wodurch die Permeabilität der Formulierungen auch bei unterschiedlichen Wirkstoffkonzentrationen verglichen werden können. Die Wirkstoffkonzentration im unteren Akzeptorkompartment wird nach Ende des Versuchs gemessen (24h bzw. 86.400 Sekunden), während die Wirkstoffkonzentration im oberen Donorkompartment der Initialkonzentration bei Start des Versuches entspricht. Die Membranfläche bei den genutzten Franzzellen ist 1 cm$^2$.

$$Papp = \left(\frac{Volumen\ Akzeptor\ in\ ml}{Membranfläche\ in\ cm^2 \times Zeit\ in\ Sekunden}\right) \times \left(\frac{Wirkstoffkonzentration\ Akzeptor}{Wirkstoffkonzentration\ Donor}\right)$$

Anbei sind die kumulierte Konzentration im unteren Kompartment nach 24h sowie die Papp Werte für die verschiedenen Formulierungen zu finden:

Kontrollformulierungen:

| Samplename | C nach 24h, kumuliert [μg/ml] | Papp [cm/sec] |
|---|---|---|
| Creme 2% Taxifolin nativ | 52,5 | 2,43x10-7 |

(fortgesetzt)

| Samplename | C nach 24h, kumuliert [μg/ml] | Papp [cm/sec] |
|---|---|---|
| Creme 0,1% Taxifolin/nur HP-β-Cyclodextrin | 11,8 | 10,9x10-7 |
| Creme 0,1% Taxifolin/nur Phospholipid | 4,7 | 4, 35x10-7 |
| Creme 0,05% Taxifolin /nur HP-β-Cyclodextrin | 6,2 | 11,5x10-7 |
| Creme 0,05% Taxifolin/nur Phospholipid | 2,6 | 4,81x10-7 |

Erfinderische Taxifolinformulierungen 0,1%:

| Samplename | C nach 24h, kumuliert [μg/ml] | Papp [cm/sec] |
|---|---|---|
| Creme 0,1% Taxifolin + HI-CAPS | 32,3 | 29,9x10-7 |
| Creme 0,1% Taxifolin + Cleargum CO 03 | 35,8 | 33,1x10-7 |
| Creme 0,1% Taxifolin + Cleargum CO 01 | 34,9 | 32,3x10-7 |
| Creme 0,1% Taxifolin + Purity Gum 2000 | 29,7 | 27,5x10-7 |
| Creme 0,1% Taxifolin + Purity Gum Ultra | 21,2 | 19,6x10-7 |
| Creme 0,1% Taxifolin + N-Creamer 2230 | 7,9 | 7,31x10-7 |
| Creme 0,1% Taxifolin + Lycoat RS 780 | 37,6 | 34,8x10-7 |
| Creme 01% Taxifolin + Lycoat RS 720 | 22,3 | 20,6x10-7 |

Erfinderische Taxifolinformulierungen (0,05% Creme):

| Samplename | C nach 24h, kumuliert [μg/ml] | Papp [cm/sec] |
|---|---|---|
| Creme 0,05% Taxifolin + HI-CAPS | 14,5 | 26,8x10-7 |
| Creme 0,05% Taxifolin + Cleargum CO 03 | 15,2 | 28,1x10-7 |
| Creme 0,05% Taxifolin + Cleargum CO 01 | 12,8 | 23,7x10-7 |
| Creme 0,05% Taxifolin + Purity Gum 2000 | 12,6 | 23,3x10-7 |
| Creme 0,05% Taxifolin + Purity Gum Ultra | 11,4 | 21,1x10-7 |
| Creme 0,05% Taxifolin + N-Creamer 2230 | 4,2 | 7,77x10-7 |
| Creme 0,05% Taxifolin + Lycoat RS 780 | 16,3 | 30,1x10-7 |
| Creme 0,05% Taxifolin + Lycoat RS 720 | 12,6 | 23,3x10-7 |

## 3. Wirkstoffformulierungen mit Naringin

Aufgrund der geringen Wasserlöslichkeit (<1mg/ml bei 25°C) sowie der schlechten Hautpenetration eignet sich Naringin ebenfalls sehr gut als Wirkstoff zur Penetrationserhöhung durch die Erfindung.

### 3.1 Material & Probenherstellung

**Material:** Naringin (98%, S3 Chemicals), Hydroxypropyl-β-Cyclodextrin (Beaute CD 110, Roquette), Phospholipide (Lipoid P 75, 70% Phosphatidylcholin/PC, Lipoid), Natriumoctenylsuccinatstärke (Hi-CAPS 100, Cleargum CO 03, Cleargum CO 01, Purity Gum 2000, Purity Gum Ultra, N-Creamer 2230), Hydroxypropylstärken (Lycoat RS 780, Lycoat RS 720), Butylene Glycol (Brontide, Genomatica), HPLC Grade Wasser.

Die Gesamtzusammensetzung im Sinne der Erfindung ist wie folgt:

| Wirkstoff | Konzentration w/w |
|---|---|
| Naringin | 0,5% |
| Hydroxypropyl-β-Cyclodextrin | 15% |
| Lipoid P 75<br>-davon Phospholipide berechnet als PC | 4,3%<br>3% |
| Modifizierte Stärke 20% w/w Lösung<br>-davon modifizierte Stärke | 3,75%<br>0,75% |
| Butylene Glycol | 15% |
| Wasser | 61,45% |

Für die Zubereitung (25g gesamt) wurden zunächst 3,75g Hydroxypropyl-β-Cyclodextrin in 15,36g Wasser unter Rühren (300rpm, 25°C) vollständig gelöst. Hierzu wurden anschließend 0,125g Naringin gegeben und unter identischen Bedingungen gerührt, bis der Wirkstoff gelöst war. Zu dieser Cyclodextrin/Naringinlösung wurden 1,075g Lipoid P 75 und 3,75g Butylene Glycol gegeben und für 30min bei 25°C und 300rpm gerührt, bis das Produkt homogen war. Zur Bildung der Liposomen wurde die Lösung hiernach bei 600 Bar für 5 Zyklen mittels Hochdruckhomogenisator (GEA, PandaPlus 1000) homogenisiert.

Anschließend wurden 0,94g einer 20%igen Stärkelösung (6 verschiedene Natriumoctenylsuccinatstärken [Hi-CAPS 100, Cleargum CO 03, Cleargum CO 01, Purity Gum 2000, Purity Gum Ultra, N-Creamer 2230] sowie 2 verschiedene Hydroxypropylstärken [(Lycoat RS 780, Lycoat RS 720]) unter Rühren hinzugegeben und die Lösung mittels Ultraschallhomogenisator (Bandelin HD 4200 Sonotrode TS 106) bei 150W für 5 Minuten homogenisiert (1s Pause, 30sec Puls).

### 3.2 Herstellung der Kontrollproben (HP-β-Cyclodextrin bzw. Phospholipid)

Zur Kontrolle bei den Penetrationsversuchen wurden darüber hinaus Wirkstoffformulierungen mit Naringin und I) nur Hydroxypropyl-β-Cyclodextrin oder II) nur Lipoid P 75/Phospholipide hergestellt. Die Gesamtzusammensetzung ist wie folgt:

I) HP-β-Cyclodextrin/Naringin

| Wirkstoff | Konzentration w/w |
|---|---|
| Naringin | 0,5% |
| Hydroxypropyl-β-Cyclodextrin | 15% |
| Wasser | 84,5% |

II) Phospholipid/Naringin

| Wirkstoff | Konzentration w/w |
|---|---|
| Naringin | 0,5% |
| Lipoid P 75<br>-davon Phospholipide | 4,3%<br>3% |
| Wasser | 95,2% |

Die Herstellung geschah gemäß der vorher beschrieben Formulierung, zunächst wurde Naringin, Wasser und die dritte Komponente (entweder Cyclodextrin oder Phospholipid) für 30min bei 25°C und 300rpm gerührt. Anschließend wurde die Lösung (im Falle der Phospholipidzugabe) mittels Hochdruckhomogenisator (GEA, PandaPlus 1000) bei 600bar in 5 Zyklen homogenisiert, um Liposomen herzustellen. Die Cyclodextrinlösung wie auch die Phospholipidlösung wurden als letzten Schritt, um Vergleichbarkeit zu der erfinderischen Formulierung zu gewährleisten, mittels Ultraschallhomogenisator (Bandelin HD 4200 Sonotrode TS 106) bei 150Wfür 5 Minuten homogenisiert (1s Pause, 30sec Puls).

### 3.3 Herstellung Cremeformulierung für Penetrationsversuche

Die Wirkstoffformulierungen nach Stärkezugabe/Homogenisation bzw. die Kontrollformulierungen wurden anschließend für die Penetrationsversuche in eine Cremeformulierung eingearbeitet. Darüber hinaus wurden auch zum Vergleich Cremeformulierungen mit reinem Naringin hergestellt.

Als Basis für die Creme wurde anionische hydrophile Creme SR genutzt, welche folgende Inhaltsstoffe enthält:

| Wirkstoff | Konzentration w/w |
|---|---|
| Emulgierender Cetylstearylalkohol (Typ A) | 21% |
| 2-Ethylhexyllaurat | 10 |
| Glycerol 85% | 4,25% |
| Kaliumsorbat | 0,14% |
| Citronensäure | 0,07% |
| Gereinigtes Wasser | 64,54% |

Zur Herstellung der finalen Formulierung wurde zunächst der Wasseranteil sowie der native Wirkstoff bzw. die wirkstoffhaltige Formulierung gemischt und diese anschließend in einem Mörser mit der Cremebasis homogenisiert. Es wurden Cremes enthaltend 1% Naringin nativ sowie enthaltend 0,05% Narinin in Form der wie oben beschrieben hergestellten erfinderischen Wirkstoffformulierung mit verschiedenen Stärken bzw. in Form der Kontrollformulierungen mit entweder nur HP-β-Cyclodextrin oder nur Phospholipid hergestellt.

Die Zusammensetzung der Cremeformulierung enthaltend natives Naringin war wie folgt:

| Wirkstoff | Konzentration w/w |
|---|---|
| Naringin nativ | 1% |
| Anionische hydrophile Creme SR | 50% |
| Wasser | 49% |

Die Zusammensetzung der Cremeformulierung enthaltend 0,05% Naringin als Wirkstoffformulierung war wie folgt:

| Wirkstoff | Konzentration w/w |
|---|---|
| Naringinformulierung<br>-davon Naringin | 5%<br>0,05% |
| Anionische hydrophile Creme SR | 50% |
| Wasser | 45% |

Es wurde jeweils eine Creme für eine erfinderische Formulierung enthaltend jede der oben besprochenen Stärken (HI-CAP 100, Cleargum CO 03, Cleargum CO 01, Purity Gum 2000, Purity Gum Ultra, N-Creamer 2230, Lycoat RS 780, Lycoat RS 720) mit 0,05% Naringinkonzentration sowie die Kontrollcremes hergestellt.

### 3.4 Penetrationsversuche mittels Franz-Diffusionszelle

Die Cremeformulierungen wurden abschließend zur Ermittlung der Hautpenetration in einer vertikalen Braunglas Franz-Diffusionszelle (PermeaGear 6 Positionen Franzzellenrührer enthaltend 6 ummantelte Franzzellen 11.28mm Durchmesser, 8ml Volumen, 1.00 cm$^2$ Membranfläche) untersucht. Hierzu wurde das Wasserbad auf 32°C beheizt, das untere Kompartment bestand aus 0,1% Tween80 in HPLC Grade Wasser, als Membran wurde die PermeaPad Skin Membran genutzt. Nach Auftragen der Cremeformulierung in das obere Kompartment wurde der Samplingarm und das obere Kompartment mit Parafilm abgedichtet. Es wurden Versuche in Triplikaten durchgeführt und die Durchschnittswerte berechnet.

Es wurden mittels einer Spritze nach 1h, 2h, 3h, 4h und 24h jeweils 300µl aus dem unteren Kompartment entnommen und mit frischer, vortemperierter Lösung (0,1% Tween80 in HPLC Grade Wasser) ersetzt. Die Samples wurden unverdünnt mittels HPLC analysiert (Agilent 1260 Infinity II DAD). Die Konzentration C im unteren Kompartment in µg/ml Naringin (1h, 2h, 3h, 4h und 24h) wurden kumuliert berechnet (unter Berücksichtigung der jeweils entnommenen 300µl).

Besonders relevant ist hierbei die kumulierte Endkonzentration im unteren Kompartment nach 24h, da hiermit die apparente Permeabilität (Papp) berechnet werden kann. Dieser Wert ist ein konzentrationsunabhängiger Maßstab für Permeabilität, wodurch die Permeabilität der Formulierungen auch bei unterschiedlichen Wirkstoffkonzentrationen verglichen werden können. Die Wirkstoffkonzentration im unteren Akzeptorkompartment wird nach Ende des Versuchs gemessen (24h bzw. 86.400 Sekunden), während die Wirkstoffkonzentration im oberen Donorkompartment der Initialkonzentration bei Start des Versuches entspricht. Die Membranfläche bei den genutzten Franzzellen ist 1 cm$^2$.

$$Papp = (\frac{Volumen\ Akzeptor\ in\ ml}{Membranfläche\ in\ cm^2 \times Zeit\ in\ Sekunden}) \times (\frac{Wirkstoffkonzentration\ Akzeptor}{Wirkstoffkonzentration\ Donor})$$

Anbei sind die kumulierte Konzentration im unteren Kompartment nach 24h sowie die Papp Werte für die verschiedenen Formulierungen zu finden:

Kontrollformulierungen:

| Samplename | C nach 24h, kumuliert [µg/ml] | Papp [cm/sec] |
|---|---|---|
| Creme 1% Naringin nativ | 11,14 | 1,03x10-7 |
| Creme 0,05% Naringin /nur HP-β-Cyclodextrin | 1,02 | 1,88x10-7 |
| Creme 0,05% Naringin/nur Phospholipid | 0,73 | 1,35x10-7 |

Erfinderische Naringin-Formulierungen (0,05% Creme):

| Samplename | C nach 24h, kumuliert [µg/ml] | Papp [cm/sec] |
|---|---|---|
| Creme 0,05% Naringin + HI-CAPS | 2,15 | 3,98x10-7 |
| Creme 0,05% Naringin + Cleargum CO 03 | 2,27 | 4,20x10-7 |
| Creme 0,05% Naringin + Cleargum CO 01 | 2,45 | 4,53x10-7 |
| Creme 0,05% Naringin + Purity Gum 2000 | 2,22 | 4,11x10-7 |
| Creme 0,05% Naringin + Purity Gum Ultra | 1,96 | 3,62x10-7 |
| Creme 0,05% Naringin + N-Creamer 2230 | 0,33 | 0,61x10-7 |
| Creme 0,05% Naringin + Lycoat RS 780 | 2,63 | 4,87x10-7 |
| Creme 0,05% Naringin + Lycoat RS 720 | 1,83 | 3,33x10-7 |

## 4. Wirkstoffformulierung mit Resveratrol

Aufgrund der geringen Wasserlöslichkeit (<0,1mg/ml bei 25°C) sowie der schlechten Hautpenetration eignet sich Resveratrol ebenfalls sehr gut als Wirkstoff zur Penetrationserhöhung durch die Erfindung.

### 4.1 Material & Probenherstellung

**Material:** Resveratrol (98%, Evolva), Hydroxypropyl-β-Cyclodextrin (Beaute CD 110, Roquette), Phospholipide (Natipide Eco, Lipoid), Natriumoctenylsuccinatstärke (Hi-CAPS 100, Cleargum CO 03, Cleargum CO 01, Purity Gum 2000, Purity Gum Ultra, N-Creamer 2230), Hydroxypropylstärken (Lycoat RS 780, Lycoat RS 720), Butylene Glycol (Brontide, Genomatica), HPLC Grade Wasser.

Die Gesamtzusammensetzung im Sinne der Erfindung ist wie folgt:

| Wirkstoff | Konzentration w/w |
|---|---|
| Resveratrol | 0,5% |
| Hydroxypropyl-β-Cyclodextrin | 15% |
| Natipide Eco<br>-davon Phospholipide berechnet als PC | 15%<br>3% |
| Modifizierte Stärke 20% w/w Lösung<br>-davon modifizierte Stärke | 5%<br>1% |
| Butylene Glycol | 15% |
| Wasser | 49,5% |

Für die Zubereitung (25g gesamt) wurden zunächst 3,75g Hydroxypropyl-β-Cyclodextrin in 12,375g Wasser unter Rühren (300rpm, 25°C) vollständig gelöst. Hierzu wurden anschließend 0,125g Resveratrol gegeben und unter identischen Bedingungen gerührt, bis der Wirkstoff gelöst war. Zu dieser Cyclodextrin/Resveratrollösung wurden 3,75g Natipide Eco und 3,75g Butylene Glycol gegeben und für 30min bei 25°C und 300rpm gerührt, bis das Produkt homogen war.

Anschließend wurden 1,25g einer 20%igen Stärkelösung (6 verschiedene Natriumoctenylsuccinatstärken [Hi-CAPS 100, Cleargum CO 03, Cleargum CO 01, Purity Gum 2000, Purity Gum Ultra, N-Creamer 2230] sowie 2 verschiedene Hydroxypropylstärken [(Lycoat RS 780, Lycoat RS 720]) unter Rühren hinzugegeben und die Lösung mittels Hochdruckhomogenisator (GEA, PandaPlus 1000) bei 450bar in 3 Zyklen homogenisiert.

### 4.2 Herstellung der Kontrollproben (HP-β-Cyclodextrin bzw. Phospholipid)

Zur Kontrolle bei den Penetrationsversuchen wurden darüber hinaus Wirkstoffformulierungen mit Resveratrol und I) nur Hydroxypropyl-β-Cyclodextrin oder II) nur Natipide Eco/Phospholipide hergestellt. Die Gesamtzusammensetzung ist wie folgt:

I) HP-β-Cyclodextrin/Resveratrol

| Wirkstoff | Konzentration w/w |
|---|---|
| Resveratrol | 0,5% |
| Hydroxypropyl-β-Cyclodextrin | 15% |
| Wasser | 84,5% |

II) Phospholipid/Resveratrol

| Wirkstoff | Konzentration w/w |
|---|---|
| Resveratrol | 0,5% |
| Natipide Eco<br>-davon Phospholipide | 15%<br>3% |

(fortgesetzt)

| Wirkstoff | Konzentration w/w |
|-----------|-------------------|
| Wasser | 84,5% |

Die Herstellung geschah gemäß der vorher beschrieben Formulierung, zunächst wurde Resveratrol, Wasser und die dritte Komponente (entweder Cyclodextrin oder Phospholipid) für 30min bei 25°C und 300rpm gerührt. Anschließend wurde die Lösung mittels Hochdruckhomogenisator (GEA, PandaPlus 1000) bei 450 bar in 3 Zyklen homogenisiert.

### 4.3 Herstellung Gelformulierung für Penetrationsversuche

Die Wirkstoffformulierungen nach Stärkezugabe/Homogenisation bzw. die Kontrollformulierungen wurden anschließend für die Penetrationsversuche in eine Gelformulierung eingearbeitet. Darüber hinaus wurden auch zum Vergleich Gelformulierungen mit reinem Resveratrol hergestellt.

Zur Herstellung der finalen Formulierung wurde zunächst das Pentylene Gylcol mit dem Gelbildner Hydroxypropylmethylcellulose (3800-5300 mPas, Carl Roth) in einem Mörser verrieben. Anschließend wurde der Wasseranteil sowie der native Wirkstoff bzw. die wirkstoffhaltige Formulierung gemischt und diese unter Rühren im Mörser mit dem verriebenen Pentylene Glycol/HPMC homogenisiert. Das Gel wurde so lange verrieben, bis keine Klumpen zu sehen waren und eine gleichmäßige Konsistenz erreicht wurde. Es wurden Gele enthaltend 1% Resveratrol nativ sowie enthaltend 0,05% Resveratrol in Form der wie oben beschrieben hergestellten erfinderischen Wirkstoffformulierung mit verschiedenen Stärken bzw. in Form der Kontrollformulierungen mit entweder nur HP-β-Cyclodextrin oder nur Phospholipid hergestellt.

Die Zusammensetzung der Gelformulierung enthaltend natives Resveratrol war wie folgt:

| Wirkstoff | Konzentration w/w |
|-----------|-------------------|
| Resveratrol nativ | 1% |
| Hydroxypropylmethylcellulose | 1% |
| Pentylene Glycol | 5% |
| Wasser | 93% |

Die Zusammensetzung der Gelformulierung enthaltend 0,05% Resveratrol als Wirkstoffformulierung war wie folgt:

| Wirkstoff | Konzentration w/w |
|-----------|-------------------|
| Resveratrolformulierung | 5% |
| -davon Resveratrol | 0,05% |
| Hydroxypropylmethylcellulose | 1% |
| Pentylene Glycol | 5% |
| Wasser | 89% |

Es wurde jeweils ein Gel für eine erfinderische Formulierung enthaltend jede der oben besprochenen Stärken (HI-CAP 100, Cleargum CO 03, Cleargum CO 01, Purity Gum 2000, Purity Gum Ultra, N-Creamer 2230, Lycoat RS 780, Lycoat RS 720) mit 0,05% Resveratrolkonzentration sowie die Kontrollgele hergestellt.

### 4.4 Penetrationsversuche mittels Franz-Diffusionszelle

Die Gelformulierungen wurden abschließend zur Ermittlung der Hautpenetration in einer vertikalen Braunglas Franz-Diffusionszelle (PermeaGear 6 Positionen Franzzellenrührer enthaltend 6 ummantelte Franzzellen 11.28mm Durchmesser, 8ml Volumen, 1.00 $cm^2$ Membranfläche) untersucht. Hierzu wurde das Wasserbad auf 32°C beheizt, das untere Kompartment bestand aus 0,1% Tween80 in HPLC Grade Wasser, als Membran wurde die PermeaPad Skin Membran genutzt. Nach Auftragen der Gelformulierung in das obere Kompartment wurde der Samplingarm und das obere Kompartment mit Parafilm abgedichtet. Es wurden Versuche in Triplikaten durchgeführt und die Durchschnittswerte berechnet.

Es wurden mittels einer Spritze nach 1h, 2h, 3h, 4h und 24h jeweils 300μl aus dem unteren Kompartment entnommen und mit frischer, vortemperierter Lösung (0,1% Tween80 in HPLC Grade Wasser) ersetzt. Die Samples wurden unverdünnt mittels HPLC analysiert (Agilent 1260 Infinity II DAD). Die Konzentration C im unteren Kompartment in μg/ml Resveratrol (1h, 2h, 3h, 4h und 24h) wurden kumuliert berechnet (unter Berücksichtigung der jeweils entnommenen 300μl).

Besonders relevant ist hierbei die kumulierte Endkonzentration im unteren Kompartment nach 24h, da hiermit die apparente Permeabilität (Papp) berechnet werden kann. Dieser Wert ist ein konzentrationsunabhängiger Maßstab für Permeabilität, wodurch die Permeabilität der Formulierungen auch bei unterschiedlichen Wirkstoffkonzentrationen verglichen werden können. Die Wirkstoffkonzentration im unteren Akzeptorkompartment wird nach Ende des Versuchs gemessen (24h bzw. 86.400 Sekunden), während die Wirkstoffkonzentration im oberen Donorkompartment der Initialkonzentration bei Start des Versuches entspricht. Die Membranfläche bei den genutzten Franzzellen ist 1 cm$^2$.

$$Papp = \left(\frac{Volumen\ Akzeptor\ in\ ml}{Membranfläche\ in\ cm^2 \times Zeit\ in\ Sekunden}\right) \times \left(\frac{Wirkstoffkonzentration\ Akzeptor}{Wirkstoffkonzentration\ Donor}\right)$$

Anbei sind die kumulierte Konzentration im unteren Kompartment nach 24h sowie die Papp Werte für die verschiedenen Formulierungen zu finden:

Kontrollformulierungen:

| Samplename | C nach 24h, kumuliert [μg/ml] | Papp [cm/sec] |
|---|---|---|
| Gel 1% Resveratrol nativ | 19,34 | 1,79x10-7 |
| Gel 0,05% Resveratrol /nur HP-β-Cyclodextrin | 3,31 | 6,12x10-7 |
| Gel 0,05% Resveratrol /nur Phospholipid | 1,82 | 3,37x10-7 |

Erfinderische Resveratrolformulierungen (0,05% Gel):

| Samplename | C nach 24h, kumuliert [μg/ml] | Papp [cm/sec] |
|---|---|---|
| Gel 0,05% Resveratrol + HI-CAPS | 8,71 | 16,1x10-7 |
| Gel 0,05% Resveratrol + Cleargum CO 03 | 8,88 | 16,4x10-7 |
| Gel 0,05% Resveratrol + Cleargum CO 01 | 9,01 | 16,6x10-7 |
| Gel 0,05% Resveratrol + Purity Gum 2000 | 8,52 | 15,7x10-7 |
| Gel 0,05% Resveratrol + Purity Gum Ultra | 6,91 | 12,8x10-7 |
| Gel 0,05% Resveratrol + N-Creamer 2230 | 1,35 | 2,50x10-7 |
| Gel 0,05% Resveratrol + Lycoat RS 780 | 9,12 | 16,9x10-7 |
| Gel 0,05% Resveratrol + Lycoat RS 720 | 7,28 | 13,5x10-7 |

## 5. Wirkstoffformulierungen mit Phloretin

Aufgrund der geringen Wasserlöslichkeit (<0,1mg/ml bei 25°C) sowie der schlechten Hautpenetration eignet sich Phloretin ebenfalls sehr gut als Wirkstoff zur Penetrationserhöhung durch die Erfindung.

### 5.1 Material & Probenherstellung

**Material:** Phloretin (98%, Symrise), Hydroxypropyl-$\beta$-Cyclodextrin (Beaute CD 110, Roquette), Phospholipide (Lipoid P 75, 70% Phosphatidylcholin/PC, Lipoid), Natriumoctenylsuccinatstärke (Hi-CAPS 100, Cleargum CO 03, Cleargum CO 01, Purity Gum 2000, Purity Gum Ultra, N-Creamer 2230), Hydroxypropylstärken (Lycoat RS 780, Lycoat RS 720), Pentylene Glycol (Dermosoft Pentiol Eco, Evonik), HPLC Grade Wasser.

Die Gesamtzusammensetzung im Sinne der Erfindung ist wie folgt:

| Wirkstoff | Konzentration w/w |
|---|---|
| Phloretin | 1% |
| Hydroxypropyl-$\beta$-Cyclodextrin | 20% |
| Lipoid P 75 -davon Phospholipide berechnet als PC | 2,85% 2% |
| Modifizierte Stärke 20% w/w Lösung -davon modifizierte Stärke | 6,25% 1,25% |
| Pentylene Glycol | 5% |
| Wasser | 64,9% |

Für die Zubereitung (25g gesamt) wurden zunächst 5g Hydroxypropyl-$\beta$-Cyclodextrin in 16,478g Wasser unter Rühren (300rpm, 25°C) vollständig gelöst. Hierzu wurden anschließend 0,25g Phloretin gegeben und unter identischen Bedingungen gerührt, bis der Wirkstoff gelöst war. Zu dieser Cyclodextrin/Phloretinlösung wurden 0,712g Lipoid P 75 und 1,25g Pentylene Glycol gegeben und für 30min bei 25°C und 300rpm gerührt, bis das Produkt homogen war. Zur Bildung der Liposomen wurde die Lösung hiernach bei 600 Bar für 5 Zyklen mittels Hochdruckhomogenisator (GEA, PandaPlus 1000) homogenisiert.

Anschließend wurden 1,56g einer 20%igen Stärkelösung (6 verschiedene Natriumoctenylsuccinatstärken [Hi-CAPS 100, Cleargum CO 03, Cleargum CO 01, Purity Gum 2000, Purity Gum Ultra, N-Creamer 2230] sowie 2 verschiedene Hydroxypropylstärken [(Lycoat RS 780, Lycoat RS 720]) unter Rühren hinzugegeben und die Lösung mittels Hochdruckhomogenisator (GEA, PandaPlus 1000) bei 450bar in 3 Zyklen homogenisiert.

### 5.2 Herstellung der Kontrollproben (HP-$\beta$-Cyclodextrin bzw. Phospholipid)

Zur Kontrolle bei den Penetrationsversuchen wurden darüber hinaus Wirkstoffformulierungen mit Phloretinl und I) nur Hydroxypropyl-$\beta$-Cyclodextrin oder II) nur Lipoid P 75/Phospholipide hergestellt. Die Gesamtzusammensetzung ist wie folgt:

I) HP-$\beta$-Cyclodextrin/Phloretin

| Wirkstoff | Konzentration w/w |
|---|---|
| Phloretin | 1% |
| Hydroxypropyl-$\beta$-Cyclodextrin | 20% |
| Wasser | 79% |

II) Phospholipid/Phloretin

| Wirkstoff | Konzentration w/w |
|---|---|
| Phloretin | 1% |
| Lipoid P 75 | 2,85% |

(fortgesetzt)

| Wirkstoff | Konzentration w/w |
|---|---|
| -davon Phospholipide | 2% |
| Wasser | 96,15% |

Die Herstellung geschah gemäß der vorher beschrieben Formulierung, zunächst wurde Phloretin, Wasser und die dritte Komponente (entweder Cyclodextrin oder Phospholipid) für 30min bei 25°C und 300rpm gerührt. Anschließend wurde die Lösung (im Falle der Phospholipidzugabe) mittels Hochdruckhomogenisator (GEA, PandaPlus 1000) bei 600bar in 5 Zyklen homogenisiert, um Liposomen herzustellen.

Die Cyclodextrinlösung wie auch die Phospholipidlösung wurden als letzten Schritt, um Vergleichbarkeit zu der erfinderischen Formulierung zu gewährleisten, mittels Hochdruckhomogenisator (GEA, PandaPlus 1000) bei 450bar in 3 Zyklen homogenisiert.

### 5.3 Herstellung Gelformulierung für Penetrationsversuche

Die Wirkstoffformulierungen nach Stärkezugabe/Homogenisation bzw. die Kontrollformulierungen wurden anschließend für die Penetrationsversuche in eine Gelformulierung eingearbeitet. Darüber hinaus wurden auch zum Vergleich Gelformulierungen mit reinem Phloretin hergestellt.

Zur Herstellung der finalen Formulierung wurde zunächst das Pentylene Gylcol mit dem Gelbildner Hydroxypropylmethylcellulose (3800-5300mPas, Carl Roth) in einem Mörser verrieben. Anschließend wurde der Wasseranteil sowie der native Wirkstoff bzw. die wirkstoffhaltige Formulierung gemischt und diese unter Rühren im Mörser mit dem verriebenen Pentylene Glycol/HPMC homogenisiert. Das Gel wurde so lange verrieben, bis keine Klumpen zu sehen waren und eine gleichmäßige Konsistenz erreicht wurde. Es wurden Gele enthaltend 2% Phloretin nativ sowie enthaltend 0,1% Phloretin in Form der wie oben beschrieben hergestellten erfinderischen Wirkstoffformulierung mit verschiedenen Stärken bzw. in Form der Kontrollformulierungen mit entweder nur HP-β-Cyclodextrin oder nur Phospholipid hergestellt.

Die Zusammensetzung der Gelformulierung enthaltend natives Phloretin war wie folgt:

| Wirkstoff | Konzentration w/w |
|---|---|
| Phloretin nativ | 2% |
| Hydroxypropylmethylcellulose | 1% |
| Pentylene Glycol | 5% |
| Wasser | 92% |

Die Zusammensetzung der Gelformulierung enthaltend 0,1% Phloretin als Wirkstoffformulierung war wie folgt:

| Wirkstoff | Konzentration w/w |
|---|---|
| Phloretinformulierung | 10% |
| -davon Resveratrol | 0,1% |
| Hydroxypropylmethylcellulose | 1% |
| Pentylene Glycol | 5% |
| Wasser | 84% |

Es wurde jeweils ein Gel für eine erfinderische Formulierung enthaltend jede der oben besprochenen Stärken (HI-CAP 100, Cleargum CO 03, Cleargum CO 01, Purity Gum 2000, Purity Gum Ultra, N-Creamer 2230, Lycoat RS 780, Lycoat RS 720) mit 0,1% Phloretinkonzentration sowie die Kontrollgele hergestellt.

### 5.4 Penetrationsversuche mittels Franz-Diffusionszelle

Die Gelformulierungen wurden abschließend zur Ermittlung der Hautpenetration in einer vertikalen Braunglas Franz-Diffusionszelle (PermeaGear 6 Positionen Franzzellenrührer enthaltend 6 ummantelte Franzzellen 11.28mm Durchmesser, 8ml Volumen, 1.00 cm$^2$ Membranfläche) untersucht. Hierzu wurde das Wasserbad auf 32°C beheizt, das untere Kompartment bestand aus 0,1% Tween80 in HPLC Grade Wasser, als Membran wurde die PermeaPad Skin Membran genutzt. Nach Auftragen der Gelformulierung in das obere Kompartment wurde der Samplingarm und das obere Kompartment mit Parafilm abgedichtet. Es wurden Versuche in Triplikaten durchgeführt und die Durchschnittswerte berechnet.

Es wurden mittels einer Spritze nach 1h, 2h, 3h, 4h und 24h jeweils 300μl aus dem unteren Kompartment entnommen und mit frischer, vortemperierter Lösung (0,1% Tween80 in HPLC Grade Wasser) ersetzt. Die Samples wurden unverdünnt mittels HPLC analysiert (Agilent 1260 Infinity II DAD). Die Konzentration C im unteren Kompartment in μg/ml Resveratrol (1h, 2h, 3h, 4h und 24h) wurden kumuliert berechnet (unter Berücksichtigung der jeweils entnommenen 300μl).

Besonders relevant ist hierbei die kumulierte Endkonzentration im unteren Kompartment nach 24h, da hiermit die apparente Permeabilität (Papp) berechnet werden kann. Dieser Wert ist ein konzentrationsunabhängiger Maßstab für Permeabilität, wodurch die Permeabilität der Formulierungen auch bei unterschiedlichen Wirkstoffkonzentrationen verglichen werden können. Die Wirkstoffkonzentration im unteren Akzeptorkompartment wird nach Ende des Versuchs gemessen (24h bzw. 86.400 Sekunden), während die Wirkstoffkonzentration im oberen Donorkompartment der Initialkonzentration bei Start des Versuches entspricht. Die Membranfläche bei den genutzten Franzzellen ist 1 cm$^2$.

$$Papp = \left(\frac{Volumen\ Akzeptor\ in\ ml}{Membranfläche\ in\ cm^2 \times Zeit\ in\ Sekunden}\right) \times \left(\frac{Wirkstoffkonzentration\ Akzeptor}{Wirkstoffkonzentration\ Donor}\right)$$

Anbei sind die kumulierte Konzentration im unteren Kompartment nach 24h sowie die Papp Werte für die verschiedenen Formulierungen zu finden:

Kontrollformulierungen:

| Samplename | C nach 24h, kumuliert [μg/ml] | Papp [cm/sec] |
|---|---|---|
| Gel 2% Phloretin nativ | 3,91 | 1,81x10-8 |
| Gel 0,1% Phloretin /nur HP-β-Cyclodextrin | 3,58 | 33,3x10-8 |
| Gel 0,1% Phloretin /nur Phospholipid | 0,55 | 5,09x10-8 |

Erfinderische Phloretinformulierungen (0,1% Gel):

| Samplename | C nach 24h, kumuliert [μg/ml] | Papp [cm/sec] |
|---|---|---|
| Gel 0,1% Phloretin + HI-CAPS | 13,98 | 129,4x10-8 |
| Gel 0,1% Phloretin + Cleargum CO 03 | 15,81 | 146,3x10-8 |
| Gel 0,1% Phloretin + Cleargum CO 01 | 16,43 | 152,1x10-8 |
| Gel 0,1% Phloretin + Purity Gum 2000 | 14,27 | 132,1x10-8 |
| Gel 0,1% Phloretin + Purity Gum Ultra | 8,16 | 75,5x10-7 |
| Gel 0,1% Phloretin + N-Creamer 2230 | 2,94 | 27,2x10-8 |
| Gel 0,1% Phloretin + Lycoat RS 780 | 16,22 | 150,2x10-8 |
| Gel 0,1% Phloretin + Lycoat RS 720 | 10,67 | 98,79x10-8 |

**Patentansprüche**

1. Zusammensetzung, umfassend Wasser und Liposomen, die mit mindestens einem Wirkstoff beladen sind, und an ihrer Außenseite eine Beschichtung aufweisen, die modifizierte Stärke umfasst,

wobei der Wirkstoff mit einem alkylierten oder hydroxyalkylierten Derivat von β-Cyclodextrin, insbesondere mit Hydroxypropyl-β-Cyclodextrin, assoziiert ist, und der Gewichtsanteil des Wirkstoffs 0,1-3 % w/w beträgt und der Gewichtsanteil des β-Cyclodextrinderivats 10-25 % w/w beträgt, jeweils bezogen auf die Gesamtzusammensetzung,

die Liposomen ein oder mehrere Phospholipide umfassen, und der Gewichtsanteil der Phospholipide bezogen auf die Gesamtzusammensetzung 1-4 % w/w beträgt,

der Gewichtsanteil der modifizierten Stärke 0,5-1,5% w/w beträgt, bezogen auf die Gesamtzusammensetzung, und

die beschichteten Liposomen einen Polydispersibilitätsindex (PDI) von < 0,3, vorzugsweise < 0,25 und besonders bevorzugt < 0,2, und eine Partikelgröße im Bereich von 100-200 nm, vorzugsweise 125-175 nm, aufweisen.

2. Zusammensetzung nach Anspruch 1, wobei der Wirkstoff einen Einschlusskomplex mit Hydroxypropyl-β-Cyclodextrin bildet.

3. Zusammensetzung nach Anspruch 1 oder 2, wobei die Liposomen einen Hohlraum aufweisen, in den der mit dem β-Cyclodextrinderivat assoziierte Wirkstoff, insbesondere der Einschlusskomplex des Wirkstoffs mit Hydroxypropyl-β-Cyclodextrin eingebettet ist.

4. Zusammensetzung nach einem der vorhergehenden Ansprüche, wobei der Wirkstoff eine Wasserlöslichkeit von < 1 mg/ml aufweist.

5. Zusammensetzung nach einem der vorhergehenden Ansprüche, wobei die Liposomen weiterhin ein oder mehrere Polyole umfassen, die vorzugsweise aus der Gruppe ausgewählt sind, die Butylenglycol, Pentylenglycol, Propylenglycol, Glycerin und Kombinationen davon umfasst, wobei der Gewichtsanteil der Polyole bezogen auf die Gesamtzusammensetzung insgesamt 2-20 % w/w, bevorzugt 5-15 % w/w betragen kann.

6. Zusammensetzung nach einem der vorhergehenden Ansprüche, wobei die Beschichtung der Liposomen

(i) modifizierte Stärke mit einen Substitutionsgrad von 0,1-10%, vorzugsweise 0,1-5%, weiter bevorzugt 0,5-3% w/w umfasst, bezogen auf die Trockenmasse, und/oder
(ii) aus einer Stärkelösung mit einer Viskosität von < 100 mPas, insbesondere < 75 mPas, bevorzugt < 50 mPas, besonders bevorzugt < 25 mPas, am stärksten bevorzugt < 15 mPas, erhalten wurde.

7. Zusammensetzung nach einem der vorhergehenden Ansprüche, wobei die modifizierte Stärke mit Octenylsuccinat-Gruppen oder Hydroxypropyl-Gruppen modifiziert ist, und der Substitutionsgrad für Hydroxypropylstärke vorzugsweise bei 0,1-5 % w/w, insbesondere 0,5-5 % w/w. oder 0,5-3 % w/w liegt und der Substitutionsgrad für Octenylsuccinatstärke vorzugsweise bei 0,1-10% w/w, insbesondere 0,5-7 % w/w liegt.

8. Zusammensetzung nach einem der vorhergehenden Ansprüche, wobei die Zusammensetzung

0,25-2 % w/w Wirkstoff, vorzugsweise 0,5-1,5 % w/w Wirkstoff,
1,5-3 % w/w Phospholipide, vorzugsweise 2-3 % w/w Phospholipide, und/oder
12,5-22,5 % w/w β-Cyclodextrinderivat, vorzugsweise 15-20 % w/w β-Cyclodextrinderivat umfasst.

9. Zusammensetzung nach einem der vorhergehenden Ansprüche, wobei die Zusammensetzung eine Zusammensetzung für die topische Verabreichung ist, insbesondere eine Creme, eine Salbe, ein Serum oder ein Hydrogel.

10. Zusammensetzung nach einem der vorhergehenden Ansprüche, wobei die Zusammensetzung eine kosmetische Zusammensetzung ist.

11. Verfahren zur Herstellung einer wässrigen Zusammensetzung von beschichteten Liposomen, die mit einem Wirkstoff, der mit alkyliertem oder hydroxyalkyliertem β-Cyclodextrinderivat assoziiert ist, beladen sind, umfassend die Schritte:

a) Herstellen einer Lösung von alkyliertem oder hydroxyalkyliertem β-Cyclodextrinderivat, insbesondere Hydroxypropyl-β-Cyclodextrin und mindestens einem Wirkstoff, um den Wirkstoff mit dem β-Cyclodextrinderivat zu assoziieren, vorzugsweise um einen Einschlusskomplex des Wirkstoffs in Hydroxypropyl-β-Cyclodextrin zu bilden,

b) Bilden von Liposomen, die einen Hohlraum umschließen in dem der mit dem β-Cyclodextrinderivat assoziierte Wirkstoff, insbesondere der Einschlusskomplex des Wirkstoffs in Hydroxypropyl-β-Cyclodextrin, eingebettet ist, und

c) Beschichten der Liposomen an ihrer Außenseite mit einer Beschichtung, die modifizierte Stärke umfasst.

12. Verfahren nach Anspruch 11, wobei die modifizierte Stärke wie in einem der Ansprüche 6 und 7 definiert ist.

13. Verfahren nach Anspruch 11 oder 12, wobei in Schritt b) Liposom-bildende Moleküle, insbesondere Phospholipide, und wahlweise Polyole wie Butylenglycol, Pentylenglykol, Propylenglycol und/oder Glycerin, zu der in Schritt a) erhaltenen Lösung zugegeben werden, vorzugsweise Phospholipide in einer Menge im Bereich von 1-4 % w/w, bezogen auf die Gesamtmasse der Zusammensetzung und optional Polyole in einer Menge im Bereich von 2-20 % w/w, bevorzugt 5-15 % w, bezogen auf die Gesamtmasse der Zusammensetzung.

14. Verfahren nach einem der Ansprüche 11-13, wobei das Beschichten der Liposomen in Schritt c) ein In-Kontakt-Bringen der in Schritt b) erhaltenen Liposomen mit einer Lösung der modifizierten Stärke und Ultraschall- oder Hochdruckhomogenisation umfasst.

15. Verfahren nach einem der Ansprüche 11-14, wobei die Stärkemenge bezogen auf die Gesamtmasse der Zusammensetzung so gewählt wird, dass sie im Bereich von 0,5-1,5% w/w liegt.

16. Verfahren nach einem der Ansprüche 11-15, zur Herstellung einer Zusammensetzung nach einem der Ansprüche 1-10.

## Fig. 1

**Fig. 2**

Stärke           Hydroxypropylstärke

Europäisches
Patentamt

European
Patent Office

Office européen
des brevets

# EUROPÄISCHER RECHERCHENBERICHT

Nummer der Anmeldung

EP 24 16 9478

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | SHAO PING ET AL: "Environmental stress stability of pectin-stabilized resveratrol liposomes with different degree of esterification", INTERNATIONAL JOURNAL OF BIOLOGICAL MACROMOLECULES, ELSEVIER BV, NL, Bd. 119, 21. Juli 2018 (2018-07-21), Seiten 53-59, XP085470274, ISSN: 0141-8130, DOI: 10.1016/J.IJBIOMAC.2018.07.139 * das ganze Dokument * ----- | 1-16 | INV. A61K9/00 A61K8/14 A61K9/127 A61K31/05 A61K31/12 A61K31/352 A61K31/7048 A61Q19/00 A61K47/69 |
| A | PARK SOO NAM ET AL: "Chitosan-coated liposomes for enhanced skin permeation of resveratrol", JOURNAL OF INDUSTRIAL AND ENGINEERING CHEMISTRY, Bd. 20, Nr. 4, Juli 2014 (2014-07), Seiten 1481-1485, XP093208033, KOREA ISSN: 1226-086X, DOI: 10.1016/j.jiec.2013.07.035 * das ganze Dokument * ----- | 1-16 | |
| A | SINSINWAR SIMRAN ET AL: "Development and characterization of catechin-in-cyclodextrin-in-phospholipid liposome to eradicate MRSA-mediated surgical site infection: Investigation of their anti-infective efficacy through in vitro and in vivo studies", INTERNATIONAL JOURNAL OF PHARMACEUTICS, ELSEVIER, Bd. 609, 29. September 2021 (2021-09-29), XP086850837, ISSN: 0378-5173, DOI: 10.1016/J.IJPHARM.2021.121130 [gefunden am 2021-09-29] * das ganze Dokument * ----- | 1-16 | RECHERCHIERTE SACHGEBIETE (IPC) A61K A61Q |

Der vorliegende Recherchenbericht wurde für alle Patentansprüche erstellt

| Recherchenort | Abschlußdatum der Recherche | Prüfer |
|---|---|---|
| München | 25. September 2024 | Nyeki, Agnes |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
.............................................................................
& : Mitglied der gleichen Patentfamilie, übereinstimmendes
    Dokument

EPO FORM 1503 03.82 (P04C03)